# EUROPÄISCHE PATENTSCHRIFT

(11) **EP 0 719 555 B1**
(45) Veröffentlichungstag und Bekanntmachung des Hinweises auf die Patenterteilung: **08.11.2000**
(21) Anmeldenummer: 95118939.8
(22) Anmeldetag: 01.12.1995
(51) Int. Cl.: A61K 31/4045, A61K 9/52, A61K 9/00

(54) **Verwendung von Melatonin zur Herstellung peroraler pulsatiler Arzneiformen**
Use of melatonin for the manufacture of oral pulsating pharmaceutical forms
Utilisation de la mélatonine pour l'obtention des formes pharmaceutiques pulsatoires à l'administration orale

(30) Priorität: 05.12.1994 DE 4443175
(43) Veröffentlichungstag der Anmeldung: 03.07.1996
(73) Patentinhaber: Jenapharm GmbH & Co. KG, 07745 Jena (DE)
(72) Erfinder: Dittgen, Michael, Prof., D-99510 Apolda (DE); Fricke, Sabine, Dr., D-07749 Jena (DE); Gräser, Thomas, Dr., D-99097 Erfurt (DE); Osterwald, Hermann, Dr., D-37120 Bovenden (DE); Oettel, Michael, Prof., D-07743 Jena (DE); Lippert, Theodor Hermann, Prof., D-72076 Tübingen (DE)
(74) Vertreter: Cramer, Eva-Maria

(56) Entgegenhaltungen:
- EP-A- 0 518 468
- WO-A-94/07487
- WO-A-95/03043
- BR. J. CLIN. PHARMACOL., Bd. 19, Nr. 4, 1985, Seiten 517-521, XP002020554 ALDHOUS, M. ET AL: "Plasma Concentrations of Melatonin in Man Following Oral Absorption of Different Preparations"
- INT. J. PHARM., Bd. 124, Nr. 1, 1995, Seiten 119-127, XP002020555 LEE, B.-J. ET AL: "Design and Evaluation of an Oral Controlled Release Delivery System for Melatonin in Human Subjects"

## Beschreibung

Die Erfindung betrifft die Verwendung von Melatonin zur Herstellung peroraler pulsatiler Arzneiformen zur Sicherung effektiver Blutwerte des Arzneistoffes, die im Vergleich zu herkömmlichen Melatonin enthaltenden Arzneiformen mit kontrollierter Freisetzung eine relativ kurze Invasionsphase realisieren und gleichzeitig die bekannten Nebenwirkungen ausschließen.
Weiterhin wird mittels Vereinfachung der Herstellung durch weniger apparativen Aufwand, eine Ersparnis spezieller Überzugstechniken ermöglicht und auch eine kostenintensive Präzisionsfertigung ausgeschlossen.

Der Terminus "Pulsatile Arzneiform" ist synonym mit den Termini "Regulierte oder modulierte Freigabesysteme" und bezeichnet nach Peppas [Peppas, N.A., Preface in Gurny, R.; Junginger, H.E.; Peppas, N.A. (Eds.) Pulsatile drug delivery, current applications and future trends, 1. Ed., S. 5 - 5, Wiss. Verlagsges., Stuttgart 1993] ein Arzneistoffabgabesystem, das in der Lage ist, als Antwort auf die Situation in der umgebenden Flüssigkeit oder auf einen äußeren Anstoß hin den enthaltenden Arzneistoff in vorgeschriebenen Intervallen freizusetzen .

Das vorzugsweise Einsatzgebiet pulsatiler Arzneiformen sind Krankheiten, wie Ischämie, Asthma, Arthritis, Schlafstörungen, Parkinson-Syndrom, Inkontinenz, Aids und Schmerzzustände [Junginger, H.E., Oral applications of pulsatile drug delivery in Gurny, R.; Junginger, H.E.; Peppas, N.A. (Eds.) Pulsatile drug delivery, current applications and future trends, Ed. 1., S. 113 - 134, Wiss. Verlagsges., Stuttgart 1993]. Diese Krankheiten oder Schmerzzustände unterliegen zeitlichen Schwankungen, so daß sie mit Arzneiformen am besten behandelt werden können, die mit der schubweisen (pulsatilen) Freisetzung des Arzneistoffes in einem Schub (one pulse system) oder in zwei Schüben (bimodal, double pulse system) einher gehen.

Typische pulsatile Arzneiformen setzen den enthaltenen Arzneistoff in einem Schub (one pulse system) oder in zwei Schüben (bimodal, double pulse system) frei.

Darüber hinaus sind jedoch auch kompliziertere Systeme und Mischformen (mixed systems) beschrieben worden, die gegebenenfalls den Arzneistoff in mehreren Schüben freisetzen können.
Als Beispiele für pulsatile Arzneiformen werden von Junginger [Junginger, H.E., Oral applications of pulsatile drug delivery in Gurny,R.; Junginger, H.E.; Peppas, N.A. (Eds.) Pulsatile drug delivery, current applications and future trends, Ed. 1., S. 1 13 - 134, Wiss. Verlagsges., Stuttgart 1993] insbesondere erwähnt:
- überzogene Tabletten, Pellets oder Mikrokügelchen,
- osmotische Systeme,
- spezielle Kapseln,
- zeitkontrollierte Explosionssysteme,
- spezielle Schichttabletten.
Die bisher bekannten Verfahren zur Herstellung einer pulsatilen Arzneiform weisen somit alle den erheblichen Nachteil auf, daß spezielle Fertigungslinien sowie aufwendige Vorrichtungen oder eine kostenintensive Präzisionsfertigung notwendig sind.

Melatonin ( N-Acetyl-5-methoxytryptamine) selbst ist ein Hormon der Zirbeldrüse und wird vorwiegend während der Nachtstunden sezerniert. Da Melatonin vielfältig in die verschiedensten Regelkreise im Körper eingreift und unterschiedliche physiologische und pharmakologische Effekte ausübt, sind aus der Fach- und Patentliteratur verschiedene-Versuche und Ergebnisse der unterschiedlichen Wirkrichtungen des Melatonins bekannt.

Beispielsweise zeigt Abbildung 1 nach STOKKAN, K.A. et al.: J.Pineal Res 16, 33-36, 1994 und PARRY, B.L. et al.: Arch.Gen.Psychatry 47, 1139-1146, 1990 in ihrer grafischen Darstellung der Blutspiegelkurve (Konzentration des Melatonin gegen die Zeit) den physiologischen Verlauf der cirkadianen Melatoninfreisetzung bei gesunden (I) und bei kranken Menschen (II) auf.

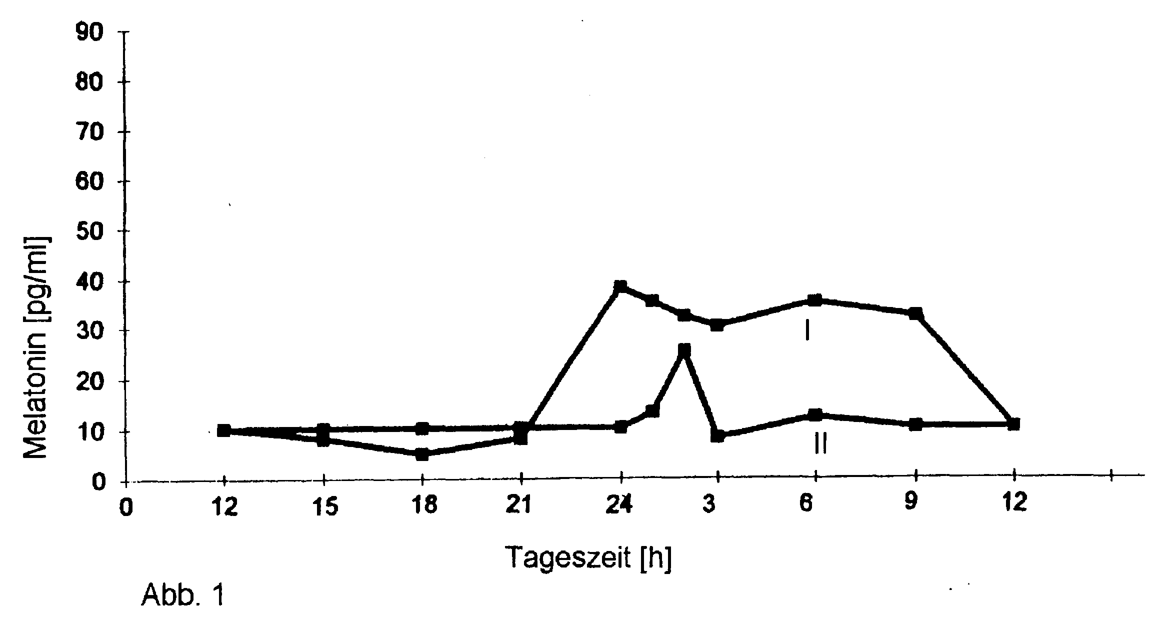

Es ist ersichtlich, daß
- bei gesunden Menschen ein relativ steiler Anstieg des Anflutens des endogenen Melatonins zu verzeichnen ist, wodurch eine schnelle Einschlafphase gekennzeichnet ist; danach erfolgt ein relativ flacher Abfall des Blutspiegels, welcher die Durchschlafphase sichert,
- bei kranken Menschen das Blutspiegelmaximum (cₘₐₓ₋ₑ) des endogenen Melatonins verspätet ist und damit verbunden die Verzögerung der Einschlafphase auftritt; danach erfolgt ein steiler Abfall des Blutspiegels - es tritt die Aufwachphase ein, die Durchschlafphase ist nicht gesichert -.

Appliziert wurde Melatonin bisher beispielsweise als intranasale, intraokulare, intravaginale oder rektale Zubereitungsform.
Damit wird zwar die Umgehung der Leberpassage realisiert, jedoch besteht der Nachteil bei diesen Lösungen darin, daß in geringen Abständen von 1 bis 2 Stunden nachdosiert werden muß.
Die Ursache dafür ist nach WETTERBERG,L. et al: A simplified radioimmunossay for melatonin and its application to biological fluids, preliminary observations on the half-life of plasma melatonin in man, Clinica Chimica Acta 86: 169-177, 1978; ALDHOUS, M. et al.: Plasma concentration of melatonin im man following oral absorption of different preparations, Br.J.Clin.Pharmac. 19: 517,521, 1985 und MALLO, C. et al.: Eur.J.Clin.Pharmacol. 38: 297-301, 1990 in der extrem kurzen Halbwertszeit von Melatonin zu sehen, die bei etwa 30 Minuten liegt.

In der WO 91/06290 werden sowohl die Herstellung als auch die Verwendungsmöglichkeiten adhäsiver Trägersysteme ("transmucosal patches") beschrieben.
Es wird darauf hingewiesen, daß diese Trägersysteme besonders gut geeignet wären, therapeutisch einen effektiven Blutspiegel herbeizuführen und/oder aufrechtzuerhalten.
In einem Ausführungsbeispiel wird die Verwendung der "patches" für die Administration von Melatonin beschrieben.
Nach SHORT R.V.: Melatonin. Hormone of darkness. Br.Med.J. 307: 952-953, 1993 erbringt die transdermale Applikation mit verschiedenartigen Pflastern keine befriedigenden Resultate, da der Melatoninanstieg im Serum sehr flach ist und kein deutlicher und abrupter Abfall nach Pflasterentfernung nachzuweisen ist.

Weiterhin bekannte Applikationen erfolgten oral, in Form von Tabletten, Dragees oder Hartgelatinekapseln.

Nachweislich kommt es nach oraler Gabe von Melatonin zu extrem hohen interindividuellen Differenzen hinsichtlich der Resorption aus dem Magen-Darm-Trakt sowie zu extrem überschießenden initialen Blutspiegeln sowie zu einer schlecht steuerbaren Wirkstoffkinetik bei wiederholter Applikation - WALDHAUSER F. et al.: Sleep laboratory investigations on hypnotic properties of melatonin, Psychopharmacology 100: 222-226, 1990 und VERMEULEN, A.: The male climacterium, Ann.Med. 25: 531-534, 1993.

Bei den Peroralia auf der Basis von Melatonin zwingt der sehr deutliche First-Pass-Mechanismus - LANE E.A. et al.: Pharmacokinetics of melatonin in man: first pass hepatic metabolism, J.Clin.Endocrinol.Metab. 61: 1214-1216, 1985 zur unphysiologisch überhöhten Dosierung.
Nachweislich werden dadurch erhöhte Krampfbereitschaft, Konfusion und Schlaflosigkeit hervorgerufen - WALDHAUSER F. et al.: Sleep laboratory investigations on hypnotic properties of melatonin, Psychopharmacology 100: 222-226, 1990 und VERMEULEN, A.: The male climacterium, Ann.Med. 25: 531-534, 1993.

Die WO 95/03043 beschreibt eine Melatoninkapsel mit modifiziertem Freisetzungsprofil, wobei die Kapsel schnell freisetzende und langsam freisetzende Bestandteile enthält.
Der Wirkstoffgehalt an Melatonin im schnell freisetzenden Bestandteil ist niedriger als im langsam freisetzenden Bestandteil.

Die WO 94/07487 beschreibt eine schnell freisetzende Kapselzubereitung, die verschieden von einer pulsatilen bimodalen Arzneiform in Form einer Kapsel ist, enthaltend Melatonin. Es wird eine Invasionsphase kleiner 2 Stunden aufgezeigt.

Der Nachteil dieser Lösung besteht darin, daß die Applikation der Arzeinform
- entweder 4 bis 17 Stunden vor der Zeit der endogenen Melatoninfreisetzung
- oder 6 bis 19 Stunden vor der normalen Schlafphase erfolgen sollte.

Dies setzt einen sehr eigenwilligen Einnahmerhytmus voraus, der sich besonders bei Alzheimer geschädigten Patienten nur sehr schwer verwirklichen läßt.
Der vorbezeichnete Einnahmerhytmus läßt einen sehr geringen Anfangsblutspiegel - Blutspiegelkonzentration, die sich unmittelbar nach der Verabreichnung des Arzneistoffes ergibt - und eine relativ lange Invasionsphase - Phase von der Applikation des Arzneistoffes bis zum Erscheinen am Wirkungsort - vermuten.
Die zugehörige Zeichnung (Figure 2) steht nicht im Einklang mit dem in der Anmeldung aufgezeigten Einnahmerhytmus.

[[EP 0518 468 A1, beschreibt eine melatoninhaltige Zubereitung, die Melatonin nach einem vorgegebenen Freisetzungsprofil - Abbildung 1 - abgibt. Diese Abbildung stellt das Profil des natürlich vorkommenden Melatonin im Serum eines gesunden Menschen dar, welches durch die Erfindung erreicht werden soll.

Es ist EP 0518 468 A1 nicht zu entnehmen, wie groß der Zeitraum zwischen der Applikation des Wirkstoffes und dem Erreichen des Profils ist. Eine Aussage zur Invasionsphase bzw. -zeit ist ebenfalls nicht gegeben.]]

Der vorliegenden Erfindung liegt die Aufgabe zugrunde, eine perorale pulsatile Arzneiform zur Verfügung zu stellen, die die vorstehend beschriebenen Nachteile nicht aufweist, das heißt insbesondere im Vergleich zu herkömmlichen, Melatonin enthaltenden Arzneiformen mit kontrollierter Freisetzung eine relativ kurze Invasionsphase realisiert.
Diese Aufgabe wird gelöst durch die Verwendung von Melatonin zur Herstellung einer peroralen pulsatilen bimodalen Arzneiform in Form einer Kapsel zur Sicherung effektiver Blutwerte des Arzneistoffes mit einer Invasionsphase kleiner 2 Stunden, wobei der Wirkstoff Melatonin in 2 Ansätzen in der Kapsel enthalten ist und Ansatz 2 verzögert zu Ansatz 1 freigesetzt wird und der Wirkstoffgehalt des Ansatzes 2 niedriger als der des Ansatzes 1 ist.
Der Wirkstoff wird in herkömmlicher Weise in pharmazeutisch verträgliche Trägerstoffe mit unterschiedlichen Dieelektrizitätskonstanten inkorporiert, diese den Wirkstoff enthaltenen Trägersubstanzen werden in einem Verhältnis entsprechend der erforderlichen Wirkstofffreigabe gemischt und diese Mischung der wirkstoffhaltigen Trägersubstanzen in die pulsatile Arzneiform gebracht.

Die Erfindung soll durch das nachfolgende Ausführungsbeispiel näher erläutert, jedoch nicht eingeschränkt werden.

Der Einfachheit halber werden nur Zweipuls-Systeme dargestellt.

Die als Ansatz 1 und Ansatz 2 gewählten Mischungen aus Trägersubstanz und Wirkstoff sind in nachfolgender Tabelle aufgeführt.

### Beispiel 1

### Kapsel mit Melatonin

| Substanz | Ansatz 1 | Ansatz 2 |
|---|---|---|
| Melatonin | 5 mg | 1,5 mg |
| Kollagen | - | 25 mg |
| Dispergator | 0,2 mg | - |
| Konservierungsmittel | - | 0,2 mg |
| Erdnußöl | 68,1 mg | - |

### Herstellung

Die im Ansatz 2 angegebene Menge Melatonin (Puls 2) wird in mikronisierter Form in Kollagenkügelchen (Durchmesser 50 ,um) eingebettet. Die Kollagenkügelchen werden gemeinsam mit der im Ansatz 1 angegeben Menge Melatonin (Puls 1) in Erdnußöl dispergiert.

### Wirkstofffreisetzung

Die Wirkstofffreisetzung wird in vitro, wie folgt, untersucht:

| | | |
|---|---|---|
| Apparatur: | gemäß DAB 10, V.5.4. | |
| | Rührgeschwindigkeit: | 300 U/min ± 2 U/min |
| | Temperatur: | 37 °C +1 °C |
| | Mediumvolumen: | 1000 ml |
| | Medium: | Wasser, 3% SDS |

### Ergebnis

Durch Variation des Verhältnisses von Melatonin in Ansatz 1 und 2 (A1, A2) werden in vitro Freisetzungsprofile erhalten (Abb. 2), die eine Steuerung der Melatoninfreisetzung von 90 % innerhalb von weniger als 2 Stunden und eine Steuerung der gesamten Melatonin freisetzung von 100 % innerhalb von 4 Stunden belegen.

Abbildung 2 stellt die in vitro Freisetzung von Melatonin aus Kapseln mit verschiedenen Mischungsverhältnissen von Ansatz 1 und Ansatz 2 dar.

Es wird aufgezeigt, daß durch die erwähnte Variation des Verhältnisses des Wirkstoffes in Ansatz 1 und Ansatz 2 eine Steuerung der Wirkstofffreigabe und damit verbunden die genaue Anpassung an Krankheits- oder Schmerzschübe bestimmter Krankheiten erfolgen kann.

Abbildung 3 zeigt die Ergebnisse einer pharmakologischen Untersuchung des Blutspiegels nach Applikation einer Melatonin enthaltenden peroralen pulsatilen Arzneiform.

Die maximalen Blutspiegel cₘₐₓ₋₁ und cₘₐₓ₋₂ beschreiben dabei die jeweils höchste Konzentration des Melatonin nach der gepulsten Arzneimittelzugabe in 2 Schüben.
Cₘₐₓ₋ₑ beschreibt den maximalen Blutspiegel nach Anfluten des endogenen Melatonin.

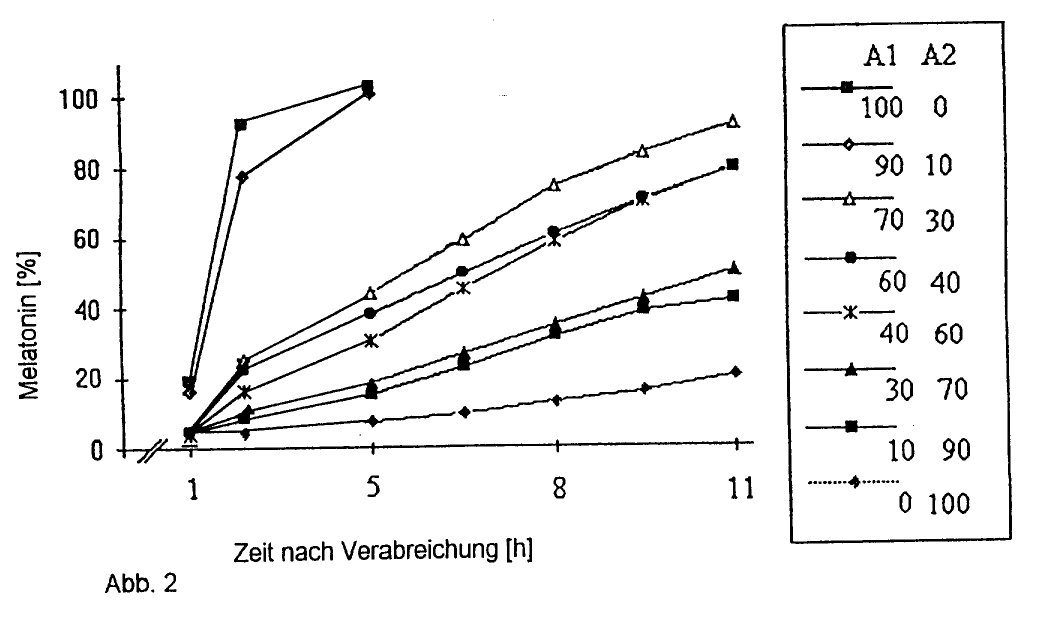

Abbildung 3 zeigt die Ergebnisse einer pharmakologischen Untersuchung des Blutspiegels nach Applikation einer Melatonin enthaltenden peroralen pulsatilen Arzneiform.

Die maximalen Blutspiegel cₘₐₓ₋₁ und cₘₐₓ₋₂ beschreiben dabei die jeweils höchste Konzentration des Melatonin nach der gepulsten Arzneimittelzugabe in 2 Schüben.
Cₘₐₓ₋ₑ beschreibt den maximalen Blutspiegel nach Anfluten des endogenen Melatonin.

Es ist ersichtlich, daß der physiologische Verlauf der Melatoninfreisetung nach peroraler pulsatiler Applikation in folgenden Schritten verläuft:
- Nach einer circa 0,5-stündigen Invasionsphase ist der erste maximale Blutspiegel, cₘₐₓ₋₁, erreicht; es wird eine schnelle Einschlafphase aufgezeigt.
- Eine bewußte Pause zum Anfluten des endogenen Melatonin - cₘₐₓ₋ₑ -wird beachtet; es tritt keine Überdosierung mit den bereits erwähnten Nebenwirkungen auf.
- Der zweite Melatonin-Schub bewirkt den zweiten maximalen Blutspiegel, cₘₐₓ₋₂, der die Durchschlafphase spezifiziert; es wird die bei kranken Menschen im Falle der cirkadianen Melatoninfreisetzung auftretende Aufwachphase zu einer Durchschlafphase kompensiert.
- Die Aufwachphase im Falle einer pulsatilen Applikation ist eine nichtverzögerte, sich über 2 Stunden erstreckende Aufwachphase, die eine "hang over" (Müdigkeit nach dem Aufwachen) vermeidet.

Der aufgeführte physiologische Verlauf der Melatoninfreisetzung, bewirkt durch die Applikation einer peroralen pulsatilen Arzneiform, für mit Schlafstörungen behaftete Menschen ist analog übertragbar auf die Steuerung der Wirkstofffreigabe und damit verbunden die genaue Anpassung an Krankheits- oder Schmerzschübe bestimmter Krankheiten, die mit dem Wirkstoff Melatonin prophylaktisch und therapeutisch behandelt werden können.

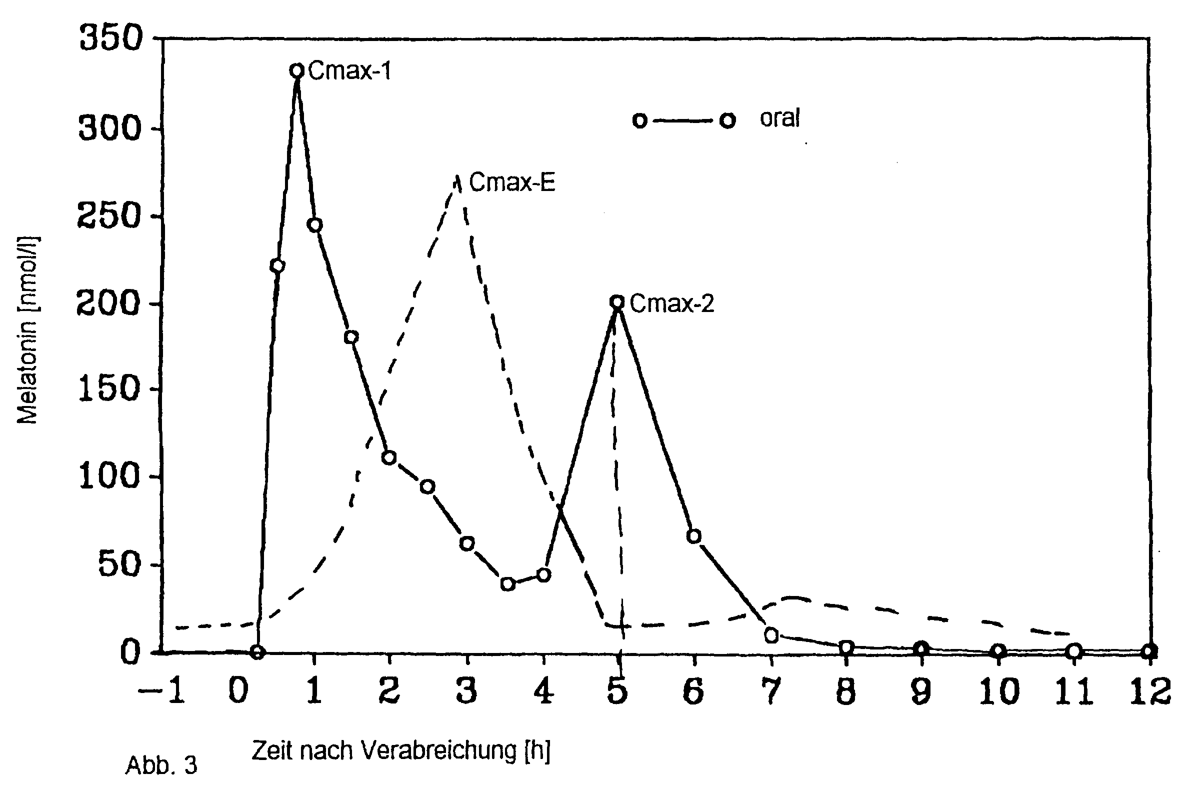

## Patentansprüche

1. Verwendung von Melatonin
zur Herstellung einer peroralen pulsatilen bimodalen Arzneiform in Form einer Kapsel zur Sicherung
effektiver Blutwerte des Arzneistoffes
mit einer Invasionsphase kleiner 2 Stunden,
wobei der Wirkstoff Melatonin in 2 Ansätzen in der Kapsel enthalten ist
und Ansatz 2 verzögert zu Ansatz 1 freigesetzt wird
und der Wirkstoffgehalt des Ansatzes 2 niedriger als der des Ansatzes 1 ist.

2. Verfahren zur Herstellung von Melatonin enthaltenden peroralen pulsatilen Arzneiformen nach Anspruch 1,
dadurch gekennzeichnet, daß man
- den Wirkstoff in herkömmlicher Weise in pharmazeutisch verträgliche Trägerstoffe mit unterschiedlichen Dieelektrizitätskonstanten inkorporiert,
- diese den Wirkstoff enthaltenen Trägersubstanzen in einem Verhältnis entsprechend der erforderlichen Wirkstofffreigabe mischt
- und diese Mischung der wirkstoffhaltigen Trägersubstanzen in die pulsatile Arzneiform bringt.

## Claims

1. Use of melatonin
for the production of a peroral pulsatile bimodal pharmaceutical form in the form of a capsule to ensure effective blood values of the pharmaceutical,
having an invasion phase of less than 2 hours,
the active compound melatonin being present in the capsule in 2 batches
and batch 2 being released with a delay relative to batch 1
and the active compound content of batch 2 being lower than that of batch 1.

2. Process for the production of melatonin-containing peroral pulsatile pharmaceutical forms according to Claim 1,
characterized in that
- the active compound is incorporated into pharmaceutically tolerable vehicles having different dielectric constants in a conventional manner,
- these vehicles containing the active compound are mixed in a ratio corresponding to the required release of active compound
- and this mixture of the active compound-containing vehicles is brought into the pulsatile pharmaceutical form.

## Revendications

1. Utilisation de la mélatonine pour la préparation d'une forme pharmaceutique orale bimodale pulsatile, sous forme d'une gélule, pour assurer des taux sanguins efficaces du principe actif avec une phase d'invasion de moins de 2 heures, dans laquelle le principe actif mélatonine est contenu en 2 charges dans la gélule et la charge 2 est libérée de façon retardée par rapport à la charge 1 et la teneur en substance active de la charge 2 est inférieure à celle de la charge 1.

2. Procédé pour la préparation de formes pharmaceutiques orales pulsatiles contenant de la mélatonine, selon la revendication 1, caractérisé en ce que
- on incorpore de façon classique la substance active dans des véhicules pharmaceutiquement acceptables, à constantes diélectriques différentes,
- on mélange ces véhicules contenant la substance active, en un rapport correspondant à la libération requise de substance active
- et on met ce mélange des véhicules, contenant la substance active, sous la forme pharmaceutique pulsatile.
